(19) Europäisches Patentamt
European Patent Office
Office européen des brevets

(11) **EP 3 470 843 A1**

(12) **EUROPEAN PATENT APPLICATION**

(43) Date of publication:
**17.04.2019 Bulletin 2019/16**

(51) Int Cl.:
***G01N 33/574*** *(2006.01)* ***G16H 50/30*** *(2018.01)*

(21) Application number: **17306403.1**

(22) Date of filing: **16.10.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Biopredictive
75007 Paris (FR)**

• **ASSISTANCE PUBLIQUE,
HOPITAUX DE PARIS
75004 Paris (FR)**

(72) Inventor: **POYNARD, Thierry
75006 PARIS (FR)**

(74) Representative: **Flesselles, Bruno F.G.
BF IP
36 rue Jean de la Fontaine
75016 Paris (FR)**

(54) **METHOD OF PROGNOSIS OF PRIMARY LIVER CANCER**

(57)    The present invention relates to new methods for assessing the risk of a patient, in particular with chronic liver disease, to develop primary liver cancer over time, using functions combining blood biochemical markers.

Figure 3

**Description**

The invention relates to a new non-invasive quantitative test that, in particular, makes it possible to detect patients susceptible to develop a liver cancer.

Primary liver cancer is a cancer of liver cells, when normal cells in the liver become abnormal and will then destroy adjacent normal tissues, and spread both to other areas of the liver and to organs outside the liver.

Most people who get liver cancer (hepatic cancer) get it in the setting of chronic liver disease. Indeed, the leading cause of liver cancer is cirrhosis due to hepatitis B, hepatitis C, and non-alcoholic (NAFLD) or alcoholic (ALD) fatty liver disease. In fact, advanced fibrosis (F2, F3 or F4 according to METAVIR classification) is present in more than 90 % of the primary liver cancer cases.

The most common types are hepatocellular carcinoma (HCC), which makes up 80% of cases, and cholangiocarcinoma. Less common types include mucinous cystic neoplasm and intraductal papillary biliary neoplasm.

The diagnosis is usually supported by medical imaging and use of some blood markers, with confirmation by tissue biopsy.

Imaging modalities include sonography (ultrasound), computed tomography (CT) and magnetic resonance imaging (MRI). Detection of a solid and hypervascularized mass greater than 2 cm with ultrasound is indicative of HCC in 95% of the cases.

Cholangiocarcinomas, occuring in the hilar region of the liver, and often present as bile duct obstruction, are usually detected by endoscopic retrograde cholangiopancreatography (ERCP), ultrasound, CT, MRI and magnetic resonance cholangiopancreatography (MRCP).

The blood marker used for diagnostic of live cancer is alfa-fetoprotein (AFP), which may be elevated in 70% of patients with liver cancer. However, detection of this protein is not very sensitive as AFP levels could be normal in liver cancer, although a rising level of AFP is generally a sign of liver cancer. One could also look for variation of blood levels of des-gamma-carboxy prothrombin, carbohydrate antigen 19-9 (CA 19-9), carcinoembryonic antigen (CEA) and cancer antigen 125 (CA125). These markers, however, are not very specific.

It is important to detect patients with a high risk of developing liver cancer, in order to be able to propose them regular surveillance. The patients would have a higher relative risk of having a primary liver cancer as compared to the global population of patients. This would thus allow early detection of cancer onset, increased chances to cure it, while decreasing morbidity, and costs of treatment. Being able to determine, in the population of patient with advanced fibrosis, those having a higher risk of developing liver cancer, to propose a regular monitoring and costly treatment of the cause (such as anti-virus C) only to these is an important public health issue.

It is however difficult to propose a detection of primary cancer liver to all patients having chronic liver disease, in view of the relatively low occurrence of such cancer in this population (around 1-2% of the patients). The possibility to segment the population in multiple classes where patients in some classes have a higher risk of having a primary liver cancer than patients of the other classes makes it possible to both reduce the number of prescribed specific medical examinations (imagery) and increase the proportion of positive cases detected in the patients examined.

The present application discloses a new test to detect whether a patient, in particular with a chronic liver disease, has a higher risk of having a primary liver cancer, using a function made by combining the measured values of various biochemical blood markers. This increased risk corresponds to a relative increased risk, i.e. to the fact that the patient is placed in a group in which the global risk of having a primary liver cancer is higher than for patients that are not in this group. In other words, it is possible to determine a threshold which defines two groups (or class) of patients (value of the function below or higher than the threshold). The relative risk of having a primary liver for patients in one of the group will thus be significantly higher than for patients in the other group. The function is quantitative, so the higher the threshold, the higher the relative risk. It is also possible to determine more than one threshold, with relative risks different in each groups (classes) thus constituted.

It is to be noted that the functions herein disclosed were obtained using a population of patients, some of which having liver cancer. It is surprising, however, that this function is not an indicator of the presence or absence of primary liver cancer in patient, but makes it possible to identify patients not having cancer yet, but that have an increased risk of having such.

The quality of a test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be acknowledged as a curve allowing prediction of the quality of a test. It is best for the AUROC to be as closed as 1 as possible, this value describing a test which is 100 % specific and sensitive.

It is reminded that

(1) sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).

(2) specificity is the probability that the diagnosis is negative in the individuals not having the phenotype sought (non-detection of true negatives): the test is negative if the patient is not suffering from the disease. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).

(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).

(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

In order to obtain a good diagnostic test, it is important to both increase specificity and sensitivity.

In developing the assays and tests as herein disclosed, the inventor increased the sensitivity of the existing tests (Fibrotest only looking at presence of fibrosis and search for AFP as a liver cancer marker). The inventor showed that a better AUROC was obtained by creating the new test as herein described, that is still increased in tests using fibrosis markers and AFP.

Generally, a diagnosis (or prognosis) method comprises

i. a step of gathering information from the patient

ii. a step of comparing said information with regards to thresholds

iii. a step of deducing, from the difference between the patient's information and the threshold, whether the patient has a specific disease, the stage of the patient's disease, or whether the patient's state will evolve to a given state.

As a matter of illustration

i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample...). The information can be presence (or absence) and/or level of specific biological markers, whether specific from the pathogenic determinant (bacterial or viral DNA/RNA), or elevated levels of patient's markers

ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease (or for patients who have been known to later evolve to a specific disease stage, for prognosis methods). Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).

iii. the last step is actually making the diagnosis (resp. determining a prognosis) *i.e.* deciding whether or not the patient has the condition sought resp. whether or not the patient will evolve to a given clinical state), taking, in particular, into account the information gathered from the patient, the thresholds as described above. The physician may also take into account other elements (such as the consistency of the information gathered or the like) to make the diagnostic.

Some methods, such as the ones disclosed in the present application, shall also include a step i.a), which comprise the steps of modifying the information obtained from the patient in order to obtain a new type of information, which is the one that is then compared to the standards in step ii. Such modification is the combination of the values of variables in a function, and obtaining an end value.

It is further to be noted that the mere measurement of the values of levels of markers in the plasma or serum of a patient and the combination thereof in an algorithm as herein disclosed is part of a method but only provides an intermediate result (an end value or index) that would then to be compared to a reference index (threshold), in order to actually be able to pose the diagnostic.

It is also to be noted that the tests herein disclosed are not "gold-standard" tests, in the sense that the output (index calculated by the formulas herein disclosed) isn't a definitive answer as to the state of the patient. Indeed, these tests are based on statistics and there may thus be false-positive or false-negative results, which is the reason why the specific experience of the physician in interpreting the index is of importance for making the prognosis and deciding which kind of follow up is to be made to ne made for each patient.

However, due to the specificity, sensitivity, positive predictive value and negative predictive value of the tests, herein provided for various thresholds of the index, these tests are of great interest in provided a help to the physician when investigating a clinical case. Consequently, step iii as disclosed above is not direct and immediate from step ii, as the physician must interpret the result from the clinical and general context to be able to reach a conclusion.

The present application thus discloses a new test that makes it possible to determine whether a patient will develop a liver cancer over a given period of time (such as in the following 5, 10 or 15 years), especially when the patient has a chronic liver disease.

The methods comprise the step of combining the values as measured from markers present in the blood, serum or plasma of said patient through a function, in order to obtain an end index, which is indicative of a "liver cancer risk class" to which belongs the patient.

This method is performed *in vitro,* or *ex vivo.*

This method is particularly interesting in that it makes it possible to manage the follow-up of the patient, and propose further liver cancer diagnosis tests (such as imaging or new specific liver cancer test) only to a fraction of patients that are the most at risk of developing cancer. For other patients, who are not at risk of having a liver cancer in a short (less than 5 years) period, the method can be repeated at a further time, in order to determine whether there is any evolution of the status (of the end value) and whether the risk remains stable or increases (whether the patient's changes risk class). This method thus makes it possible to detect primary liver cancer at early stage and starts treatment, thus reducing mortality, morbidity and associated costs.

Below are described a few functions that can be used for performing the method herein disclosed. It is however to be noted that there is no technical difficulty to obtain and develop other functions that would be as (or more) efficient as the functions herein disclosed, following the teachings of the invention.

It is also to be noted that some functions herein disclosed only makes use of the blood (or plasma or serum) measured values of classical liver markers (fibrosis markers) and don't use the values of liver cancer markers. It is surprising to note that such prognosis of occurrence of cancer can be made without using any cancer-specific marker. This is also very useful, as the "classical" (not comprising liver cancer markers) markers are widely studied by physicians in the art. The use of a function that doesn't comprise liver-cancer markers thus makes it possible for the physician to first assess the risk of the patient and then to request a further blood test to measure the value of the liver cancer marker and refine the test, using the other function herein disclosed.

It is also to be noted that, although the function herein exemplified have been obtained by logistic regression, other statistical methods could be used to obtain the risk of developing primary liver cancer, in particular Cox regression (the hazard being the occurrence of primary liver cancer), which would thus provide other functions having the same kind of output of the logistic regression functions herein exemplified (measure the risk of primary liver cancer occurrence).

Although any markers can be used in the disclosed function, one can choose not to use bilirubin (total bilirubin) an/or transferases (ALT (Alanine Aminotransferase) or AST (Aspartate Aminotransferase)). This is because such markers may widely vary for multiple reasons.

*A contrario,* it is preferred when the function uses the levels of Haptoglobin and of Apolipoprotein A-I (apoA1).

The invention thus relates to a method for determining whether a patient, in particular presenting chronic liver disease, has a risk of developing a primary liver cancer, comprising the step of combining the values of blood markers as measured in the blood, serum or plasma of the patient through a function combining the values of the blood markers.

It is thus possible to obtain an end value, and to compare this end value to a predetermined threshold wherein the patient has a risk of developing a primary cancer liver if the end value is higher/lower than a predetermined threshold.

Indeed, the invention would thus relates to a method for determining whether a patient has a risk of developing a primary liver cancer, comprising the step of combining the values of biochemical markers as measured in the blood, serum or plasma of the patient through a function, obtaining an end value, comparting the end value to predetermined values (thresholds), wherein the variation of the end value to the predetermined values indicates the risk of the patient to develop a primary cancer liver

As indicated above, the risk shall be obtained for a given period of time after the test is made, that can span up to 15 years. The figures of the application, although illustrative and pertaining to specific functions, can be used to determine, for any duration up to 15 years, the percentage, in each class of primary liver cancer occurrence and hence the risk of each class. In particular, the risk can be assessed for 5, 10 or 15 years.

The patient preferably has a chronic liver disease, which is preferably selected from the group consisting of infection with the hepatitis B virus, infection with the hepatitis C virus, Non-Alcoholic Fatty Liver disease (NAFLD), Alcoholic liver disease (ALD). The patient may also have NASH disease (Non-alcoholic steatohepatitis).

The blood markers, the amount of which is measured and used in the function, are preferably of liver fibrosis blood markers. This corresponds to blood markers which vary when the patient has a liver disease (fibrosis generally appearing when such disease is present).

The markers can thus be selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin.

Change of the concentration of these markers is associated with nonspecific liver injury and these are thus not specifically associated with primary liver cancer.

In a preferred embodiment, the biochemical markers are selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, cytokeratin 18, platelets number, prothrombin level, hyaluronic acid, amyloid A (SAA), hemopexin and carbohydrate deficient transferrin. This group would also comprise the fucosylated forms of proteins and in particular fucosylated A2M, fucosylated haptoglobin, fucosylated apoA1.

As indicated above, it is also possible to use at least one marker of liver cancer such as $\alpha$-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-L-fucosidase (AFU), Des-$\gamma$-carboxyprothrombin (DCP or PIVKA), Osteopontin (OPN), or Human Carbonyl Reductase. Other hepatocellular carcinoma (HCC) markers are disclosed in particular in Zhao et al Mol Clin Oncol. 2013 Jul; 1(4): 593-598, Salloom, Int J Health Sci (Qassim). 2016 Jan; 10(1): 121-136, Tara Behne and Copur, International Journal of Hepatology, vol. 2012, Article ID 859076, 7 pages. Change of concentration of these markers is correlated with primary cancer liver (these markers could thus be considered as specific of liver cancer).

It is to be noted that $\alpha$-fetoprotein (AFP) is a protein that can be fucosylated or not, and has three glycoforms (AFP-L1, AFP-L2 and AFP-L3), named according to their binding ability to the lectin lens agglutinin (LCA). It is possible to detect any form of the AFP protein, alone or combinations of such isoforms. In particular, combined detection of AFP and AFP-L3 (the *Lens culinaris* agglutinin-reactive fraction of alpha-fetoprotein) can be made.

It is thus possible use a function that uses the values of serum markers of liver disease as indicated above and of at least one cancer marker.

It is also possible to include, as variable in the function, at least one other variable chosen in the group consisting of gender, age and BMI (Body Mass Index) of the patient. Fasting glucose levels could also be used.

As indicated above, the function that can be used in the method herein disclosed can be a function obtained by logistic regression (which can be called logistic function).

Methods to perform logistic regressions are known in the art. In summary, they consist in evaluating the individual variations of markers between populations of subjects (one with the output such as the liver cancer and the other where the output is absent). The markers which are the most variable can be chosen and logistic regression analysis is made to ponder the independent discriminative value of the selected markers. If some markers don't vary between groups, the coefficients of these markers in the function will be low (thereby indicating that the weight of these markers is of no real influence for the presence of the trait).

The function can then be normalized (for example to have the end result always comprised between 0 and 1).

In the present invention, the function can be obtained by any of the methods disclosed below, and in particular by

a) evaluating the presence of liver cancer after a given duration of time in a cohort of patients, preferably presenting chronic liver disease, wherein the values of the blood markers variables used in the function are known for the patients at the beginning of the duration of time

b) identifying by unidimensional analysis, among the blood markers, the value of which has been measured, the ones for which the values differ significantly between the groups of

i. patients with liver cancer and
ii. patients without liver cancer

c) performing a logistic regression analysis to assess and ponder the independent discriminative value of the markers identified in step b) for the occurrence of liver cancer in the given duration of time

d) thereby obtaining the function, by combination of these identified independent factors.

In order to obtain a function that is as accurate as possible, the number of patients in the cohort should be as large as possible, indicating that it preferably comprises more than 50 patients, preferably more than 100 patients, preferably more than 200 patients, more preferably more than 500 patients, or even more than 1000 patients. As indicated, there is no upper limit for the number of patients, and the larger, the better.

The duration of time is chosen by the person skilled in the art. If one desires that the function is indicative of the occurrence of cancer liver at five years, the duration of time shall be 5 years. This means that the groups of b) will be the patients with liver cancer after 5 years and the patients without liver cancer after 5 years. Other time periods can be used (10, 15 years or more). In the present application, the disclosed functions have been designed with a 15 years period. It is striking to note that these functions are nevertheless indicative of a primary liver cancer risk at lower duration of time (5 and 10 years). However, other functions could have been designed by the inventors, with other endpoint durations, which may be more accurate for these specific endpoints,

In particular, the inventor has shown that it is possible to obtain an accurate function, using the values measured for alpha-2-macroglobulin (A2M), apoA1, GGT and haptoglobin. In a more specific embodiment, the function doesn't include the values of other markers of blood, serum or plasma. In other words, the only values of blood, serum or plasma markers that are used within the logistic function are the values measured for apoA1, A2M, GGT and haptoglobin.

The function may further include at least one other variable chosen in the group consisting of gender, age and BMI of the patient. In this embodiment, the logistic function will use both the values of the markers from blood, serum or plasma and the value measured from at least one of the other variable as recited above. It is preferred when the logistic function further takes into consideration both the gender (sex) and the age of the patient.

Consequently, a function, as usable in the context of the present invention, may have the form:

$$F1 = a_0 + a_1 \times Log\,(A2M,\,g/l) + a_2 \times Age\,(years) + a_3 \times ApoA1\,(g/l) + a_4 \times$$
$$Gender\,(0\ for\ women,\ 1\ for\ men) + a_5 \times Log(GGT,\,IU\,/\,l) + a_6 \times Log\,(Hapto,\,g/l).$$

In this embodiment,

- $-6 \leq a_0 \leq -3.4$ preferably $-5.2 \leq a_0 \leq -4.7$
- $2.4 \leq a_1 \leq 4.6$ preferably $3 \leq a_1 \leq 4$
- $0.02 \leq a_2 \leq 0.07$ preferably $0.03 \leq a_2 \leq 0.07$
- $-2.6 \leq a_3 \leq -0.8$ preferably $-2.2 \leq a_3 \leq -1.0$
- $-1.5 \leq a_4 \leq -0.5$ preferably $-1.2 \leq a_4 \leq -0.6$
- $0.9 \leq a_5 \leq 1.9$ preferably $1.2 \leq a_5 \leq 1.7$
- $-1.5 \leq a_6 \leq -0.5$ preferably $-1.1 \leq a_6 \leq -0.6$.

In a preferred embodiment, the function is

$$F1\text{-}a = -4.819 + 3.673 \times LogA2M\,(g/L) + .053 \times Age\,(years) - 1.983 \times ApoA1$$
$$(g/L)\ -1.122 \times Sex\,(0\ for\ women,\ 1\ for\ men) + 1.603 \times LogGGT\,(IU/L) - 0.834 \times$$
$$LogHapto\,(g/L).$$

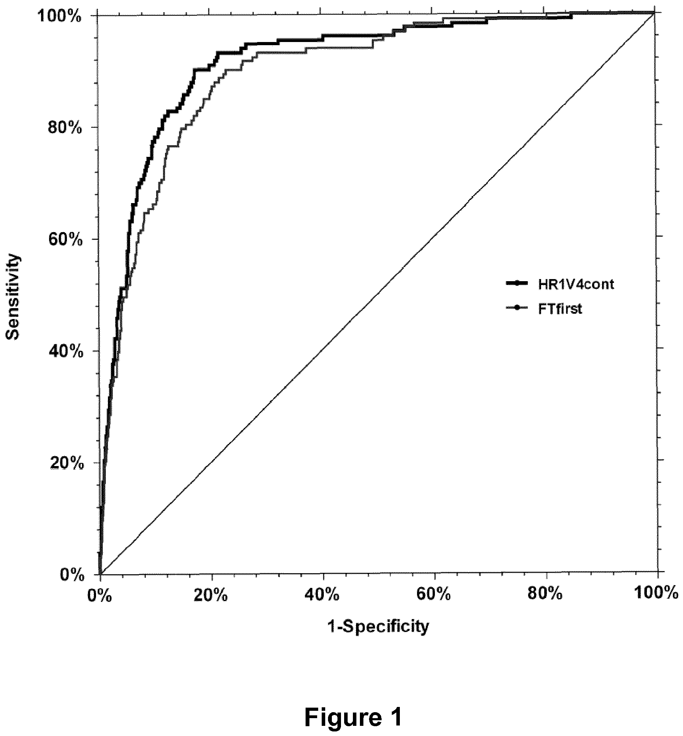

Such function for the diagnosis of contemporaneous liver cancer (current presence of liver cancer) has an AUROC value of 0.915 vs 0.896 for the FibroTest AUROC, an AUROCs difference of 0.019 (95% CI; 0.005-0.033 ; P=0.0001), i.e. a 2% difference and increase of both specificity and sensitivity (Figure 1).

In another embodiment, the function is:

$$F1\text{-}b = -4.982 + 3.713 \times \text{LogA2m (g/L)} + 0.0473 \times \text{Age (years)} - 1.133 \times \text{ApoA1 (g/L)} - 0.791 \times \text{Sex (0 for women, 1 for men)} + 1.343 \times \text{LogGGT (IU/L)} - 1.062 \times \text{LogHapto (g/L)}.$$

The function herein disclosed will provide an end result (index or score) that is compared to a threshold to determine the class of patients to which the patient belongs.

As an illustration, for function F1-a, if the end result is below 0.25, the patient belongs to the class "low cancer risk), indicating that the risk of having a primary liver cancer is 0.9792 (there would be 208 primary liver cancer cases in a population of 10000 patients having an index below 0.25).

If the end result is higher than 0.25, the patient belongs to the class "high cancer risk), indicating that the risk of having a primary liver cancer is 0.8449 (there would be 1551 primary liver cancer cases in a population of 10000 patients having an index above or equal to 0.25).

The relative risk between the two classes is thus more than 7 times higher in the group of patients having an index higher or equal to 0.25.

Consequently, in this case, setting the threshold as 0.25 makes it possible to detect patients belonging to a group with an increased risk to develop a primary liver cancer, said group consisting of patients having an end result is higher or equal to 0.25.

It is also possible to set up other thresholds and thus design other groups (classes) of patients with an increased risk to develop a primary liver cancer. As an illustration, one can design groups of patients having an end result is higher or equal to 0.25 and lower than 0.50, or higher or equal to 0.50 and lower than 0.75, or greater or equal to 0.75.

If the end result is higher than 0.25 and lower than 0.50, the patient belongs to the class "moderate cancer risk", indicating that the risk of having a primary liver cancer is 0.9124 (there would be 876 primary liver cancer cases in a population of 10000 patients having an index above or equal to 0.25 and lower than 0.50).

If the end result is higher than 0.50 and lower than 0.75, the patient belongs to the class "intermediate cancer risk", indicating that the risk of having a primary liver cancer is 0.8225 (there would be 1,775 primary liver cancer cases in a population of 10000 patients having an index above or equal to 0.25 and lower than 0.50). The relative risk in this class is thus twice the risk of the "moderate cancer risk" class.

If the end result is higher than 0.75, the patient belongs to the class "very high cancer risk", indicating that the risk of having a primary liver cancer is 0.6933, meaning that there would be 3067 primary liver cancer cases in a population of 10,000 patients having an index above or equal to 0.75 (a relative risk of more than 14 with regards to the "low cancer risk" class).

It is also possible to design and use functions where primary liver cancer markers are also used. In particular, the inventor showed that addition of AFP to the other markers makes it possible to design functions as or more efficient than functions that don't incorporate these markers. It is also to be noted that the nonspecific markers and the specific cancer markers can be combined in the same function or used in independent juxtaposition analysis.

Thus, a function may be:

$$F2 = a0 + a1 \times \text{Log (A2M, g/l)} + a2 \times \text{Age (years)} + a3 \times \text{ApoA1 (g/l)} + a4 \times \text{Gender (0 for women, 1 for men)} + a5 \times \text{Log(GGT, IU / l)} + a6 \times \text{Log (Hapto, g/l)} + a7 \times \text{Log AFP (µg/L)}.$$

In such function,

    a) $-7 \leq a0 \leq -5.5$ preferably $-6.5 \leq a0 \leq -6$
    b) $2.2 \leq a1 \leq 3.2$ preferably $2.5 \leq a1 \leq 2.9$
    c) $0.02 \leq a2 \leq 0.06$ preferably $0.04 \leq a2 \leq 0.05$
    d) $-1.65 \leq a3 \leq -1.25$ preferably $-1.55 \leq a3 \leq -1.35$
    e) $-0.3 \leq a4 \leq -0.22$ preferably $-0.28 \leq a4 \leq -0.24$
    f) $1.25 \leq a5 \leq 1.85$ preferably $1.45 \leq a5 \leq 1.65$

g) -0.75 ≤ a6 ≤ -0.55 preferably -0.7 ≤ a6 ≤ -0.6
h) 1.3 ≤ a7 ≤ 1.9 preferably 1.5 ≤ a7 ≤ 1.8

The function may be

$$F2\text{-}a = -6.214 + 2.713\ LogA2m\ (g/L) + 0.0447 \times Age\ (years) - 1.451 \times$$
$$ApoA1\ (g/L) - 0.260 \times Sex\ (0\ for\ women,\ 1\ for\ men) + 1.557 \times LogGGT\ (IU/L) -$$
$$0.633 \times LogHapto\ (g/L) + 1.662 \times LogAFP\ (\mu g/L).$$

As indicated above the function may be a (multivariate) Cox function. Such Cox function is preferably obtained by

a) Assessing / evaluating the presence of liver cancer after a given duration of time in patients presenting chronic liver disease, wherein the values of the variables used in the function are known at the beginning of the duration of time
b) Performing a multivariate Cox regression by combination of said values, on the basis of the occurrence of liver cancer after the given duration of time
c) Assessing the independent discriminative value of the values by analysis of the multivariate Cox regression,
d) Combining the relative weight of the values of each marker, as individually determined in the multivariate Cox regression, with a negative sign when the markers harbor a negative correlation with the observation of liver cancer occurrence.

The markers that can be used in this method are the same as disclosed above. The period of time is chosen by the one of skill in the art, and is generally multiple years (such as 5, 10 15 years).
Although the present application discloses a function that can be used in the methods herein proposed, the present invention also relates to a method for obtaining functions that can assist a physician to determine whether a patient, in particular with chronic liver disease is at risk of developing a primary liver cancer wherein said logistic function combines the values of the concentration of biochemical markers in the serum of said patient. Thus, depending on the markers that are chosen by the person skilled in the art, other functions than the ones herein disclosed can be obtained.
The art already describes different means making it possible to obtain a function by logistic regression, the end value of which is indicative of the degree of liver fibrosis. In particular, one can cite WO 2002/016949, and different declinations of the method first described in WO 2002/016949: WO 2010/149767, WO 2006/10357, WO 2006/082522, WO 2003/073822, WO 2011/039321, WO 2005/116901, WO 2010/058295, and WO 2010/097472.
In the present case, the end value of the function obtained by the method disclosed below is indicative of the risk for the patient to have a primary liver cancer.
The invention thus relates to a method for obtaining a function that can assist a physician to determine whether a patient, in particular with chronic liver disease is at risk of developing a primary liver cancer after a given period of time, wherein said function combines the values of the concentration of biochemical markers in the blood / serum or plasma of said patient and optionally the age, gender and BMI, comprising the steps of:

a) classifying patients of a cohort of patients into two groups according to the presence or absence of primary liver cancer after a given period of time
b) identifying by unidimensional analysis, the biochemical markers, the value of which has been measured in the patients at the beginning of the given period of time, the ones which differs significantly between the group of patients in which cancer has occurred and the group of patients in which cancer has not occurred
c) performing a logistic regression analysis to assess the independent discriminative value of these markers identified in step b) for the occurrence of primary liver cancer
d) obtaining the function by combination of these identified independent factors.

In another embodiment, the invention thus relates to a method for obtaining a function that can assist a physician to determine whether a patient, in particular with chronic liver disease is at risk of developing a primary liver cancer wherein said function combines the values of the concentration of biochemical markers in the blood / serum or plasma of said patient and optionally the age, gender and BMI, comprising the steps of:

a) classifying patients of a cohort of patients into two groups according to the presence or absence of primary liver cancer in the patients
b) identifying by unidimensional analysis, the biochemical markers, the value of which has been measured in the patients, the ones which differs significantly between the group of patients having primary liver cancer and the group of patients not having primary liver cancer

c) performing a logistic regression analysis to assess the independent discriminative value of these markers identified in step b) for the occurrence of primary liver cancer

d) obtaining the function by combination of these identified independent factors.

This method differs from the one disclosed just before, that the function is here designed on contemporaneous liver cancer, where it there is designed on primary liver cancer as they appear during the given period of time.

For both methods, the thresholds making it possible to classify the patients within risk classes are determined by classical statistical methods.

The functions herein disclosed have been designed on the basis of the second method (using contemporaneous cancers) and have proven to be predictive of the risk of developing cancer in the future, as shown in the examples. The fact that such test is predictive is thus unexpected, as it could have been expected that the function would be indicative of the contemporaneous presence of liver cancer, but it could not have been predicted that the function would be able to predict future occurrence (more than one year after performing the test) of liver cancer.

It is preferred when the biochemical markers of step b) are selected from the group consisting of α2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin.

It is preferred when the biochemical markers of step b) are selected from the group consisting of α2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, cytokeratin 18, platelets number, prothrombin level, hyaluronic acid, amyloid A (SAA), hemopexin and carbohydrate deficient transferrin. This group would also comprise the fucosylated forms of proteins and in particular fucosylated A2M, fucosylated haptoglobin, fucosylated apoA1.

It is also possible to use at least one marker of liver cancer such as α-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), α-L-fucosidase (AFU), Des-γ-carboxyprothrombin (DCP or PIVKA), Osteopontin (OPN), or Human Carbonyl Reductase, for obtaining the function.

It is preferred when one selects at least or exactly two, at least or exactly three, at least or exactly four, at least or exactly five or at least or exactly six of these markers, although there is no upper limit in the number of markers that can be used in the described methods.

It is however preferred to use a relatively small number of markers, in order to reduce the costs of performing the prognosis method (reduce the cost of blood analysis). Consequently, using three, four or five markers is preferred.

The function may further include at least one other variable chosen in the group consisting of gender, age and BMI of the patient. Fasting glucose level could also be used. However, one will rather use gender and age, as these are easy to determine, while there might be some variability in the BMI or fasting glucose (the patient may not provide the right weight or have eaten before blood harvest).

In another embodiment, it is possible to obtain the function which combines the values of the concentration of biochemical markers in the blood / serum or plasma of said patient and optionally the age, gender and BMI, comprising the steps of:

a) classifying patients of a cohort of patients into two groups according to the presence or absence of primary liver cancer in the patients, after a given period of time

b) providing

i. the indexes (or scores) obtained for the patients, at the beginning of the given period of time, wherein the indexes method as indicated above to obtain a first function combining the values of the concentration of biochemical markers in the blood / serum or plasma of said patient and optionally the age, gender and BMI, wherein the biochemical markers preferably do not comprise an marker for primary liver cancer

ii. the value of the level of a marker for primary liver cancer, at the beginning of the given period of time

c) performing a logistic regression analysis to assess the independent discriminative value of the variables i. and ii. of step b) for the occurrence of primary liver cancer

d) obtaining the function by combination of these variables.

Alternatively, it is possible to obtain the function which combines the values of the concentration of biochemical markers in the blood / serum or plasma of said patient and optionally the age, gender and BMI, comprising the steps of:

a) classifying patients of a cohort of patients into two groups according to the presence or absence of primary liver cancer in the patients

b) providing

i. the indexes (or scores) obtained for the patients, wherein the indexes method as indicated above to obtain a first function combining the values of the concentration of biochemical markers in the blood / serum or plasma of said patient and optionally the age, gender and BMI, wherein the biochemical markers preferably do not comprise an marker for primary liver cancer

ii. the value of the level of a marker for primary liver cancer

c) performing a logistic regression analysis to assess the independent discriminative value of the variables i. and ii. of step b) for the occurrence of primary liver cancer

d) obtaining the function by combination of these variables.

It is preferred when the biochemical markers of that were used to obtain the function of used in b)i. are selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin, and more preferably selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, cytokeratin 18, platelets number, prothrombin level, hyaluronic acid, amyloid A (SAA), hemopexin and carbohydrate deficient transferrin. This group would also comprise the fucosylated forms of proteins and in particular fucosylated A2M, fucosylated haptoglobin, fucosylated apoA1.

The at least one marker of liver cancer of b)ii. is preferably selected from the group consisting of $\alpha$-fetoprotein (AFP), fucolsylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-L-fucosidase (AFU), Des-$\gamma$-carboxyprothrombin (DCP or PIVKA), Osteopontin (OPN), and Human Carbonyl Reductase.

In the above methods, the cohort of patients should contain a number of patients high enough so as to ensure robust statistical analysis. It would thus contain at least 50, more preferably at least 100, ore preferably at least 250, more preferably at least 500 patients.

In the above methods, the combination of step d) is made using the coefficients calculated in step c), which depend on the discriminative values as identified in step c). Other steps can be performed, such as normalization of the function to maintain the end results within two selected boundaries.

The method herein disclosed thus makes use of an *ex vivo* combination of value of different variables, in order to obtain an end-result that is indicative of the risk of primary liver cancer occurrence. It would thus exclude any step applied on the human body of the patient. In the method herein disclosed, it is understood that the markers, the value of which is measured and used as variable in the function, are circulating proteins or natural elements that are present in the blood, plasma or serum of a patient. The values of the chosen markers shall be measured on a sample of blood, plasma or serum previously harvested, according to methods known in the art. The values are expressed in the units according to the art. However, should other units be chosen by the person skilled in the art to expressed the measured values, this would only change the coefficients within the logistic function. The method would thus still be applicable.

However, in another embodiment, the invention relates to a method for prognosis of the occurrence of primary liver cancer in a patient (in particular with chronic liver disease), comprising the step of harvesting blood, plasma or serum from a patient, measuring the value of markers present in the harvested sample and combining the values as measured through a function as disclosed above. The markers are as disclosed above.

The method and functions as herein disclosed may be used to determine the individual risk of a patient to develop PLC (primary liver cancer) during the follow-up of its chronic liver disease. It is thus usually used when the patient already presents a chronic liver disease. It could also be used during routine follow up, so as to provide an assistance for the physician to decide to start a monitoring through imaging, or to treat earlier the patient by a costly antiviral drug usually only prescribed for severe stages of chronic hepatitis C.

The invention also relates to a device for the prognosis of the risk of a patient to have a primary liver cancer, comprising a first means, wherein the first means provides an index by combining the values as measured from markers present in the serum or plasma of a patient (and optionally age and sex) through a function.

The functions and markers to be implemented within this device are the ones disclosed in the present specification. As herein disclosed the device preferably makes it possible to obtain the index using the functions F1, and in particular F1-a or F-1 b disclosed above.

In another embodiment, the device makes it possible to obtain the index using the functions F2, and in particular F2a disclosed above.

In another embodiment, the invention relates to a microprocessor comprising a computer algorithm to perform a method of determining the risk of a patient to have a primary liver cancer: providing the values of blood markers of a patient and optionally the age and a value allocated to the sex of the patient; and performing a mathematical function (as disclosed above, or obtained as disclosed above) to combine the values to obtain a score useful for the prognosis of the risk of primary liver cancer occurrence in the patient.

The invention also relates to the use of the functions, devices and/or microprocessors in order to make a diagnosis of presence of a primary liver cancer in a patient by use of the functions and comparing the result to a predetermined threshold to determine the presence of the primary liver cancer in the patient.

With the results of the function, the physician would be able to propose a personalized follow-up to patients, depending on their risk to have a liver cancer.

If patients are F2 or F3 (METAVIR classification) and the score of the test is below 0.25, patients would generally be seen again 3 to 4 years later, and new tests (Fibrotest, Liver cancer test) would be made again.

If patients are F4 (METAVIR, indicating presence of cirrhosis) and the score is <0.25, the normal follow-up would be made (every 3 to 6 months).

If the score is >=0.25, there is a risk of liver cancer. Surveillance on the basis of imaging (at intervals determined by the physician) would thus be proposed, in order to have early detection of cancer occurrence, and treat it early to limit the risks of relapses, mortality and morbidity. Imaging can be proposed to people with higher risk, so as to avoid making clinical exams having low added value (avoiding having a lot of negative results for very few positive results). By only using patient belonging to the risk class, one can increase the proportion of positive cases that are detected. It is also to be noted that the physician may decide to propose, on a regular basis, new blood tests to people in the risk class but having a score close to 0.25 (such as a score between 0.25 and 0.50) and start imaging only if the score is above 0.50 (this number being given as an illustration, the actual number being chosen by the physician).

It is also to be noted that the thresholds herein provided are only for the functions disclosed in details in this application, and that other threshold would be used for other functions.

The invention thus also relates to a method for treating a patient in need thereof, comprising the steps of:

1. Performing the methods are herein disclosed to identify whether the patient has a primary liver cancer, or has a high risk of developing a primary liver cancer, and

2. Applying a treatment regimen to the patient in need thereof according to the presence of primary liver cancer, or the risk of developing primary liver cancer.

The treatment regimen of step 2) may be one or a combination of:

- Performing surgery or non surgical methods (radio-frequency, intra arterial chemo-embolization...) to remove cancerous mass from the liver
- Providing antitumoral drugs to the patient
- Performing imagery
- Setting up a schedule for follow-up of the patient.
- Providing very early treatment of the chronic liver disease such as direct antiviral agents in CHC or NAFLD-treatment.

In summary, the present methods provide an easy-to-use blood test making it possible to identify patients with a very high risk of liver cancer, so that they can state-of-the-art imaging techniques that are able to localize very small cancers.

The following examples are meant to describe an aspect of invention, but shall not be limiting the invention.

## DESCRIPTION OF THE FIGURES

Figure 1: Compared AUROC (Area Under Receiving Operator Curve) for detection of contemporaneous primary liver cancer from function F1-a (HR1V4) and Fibrotest (FTfirst) (disclosed as f5 in WO 02/16949)

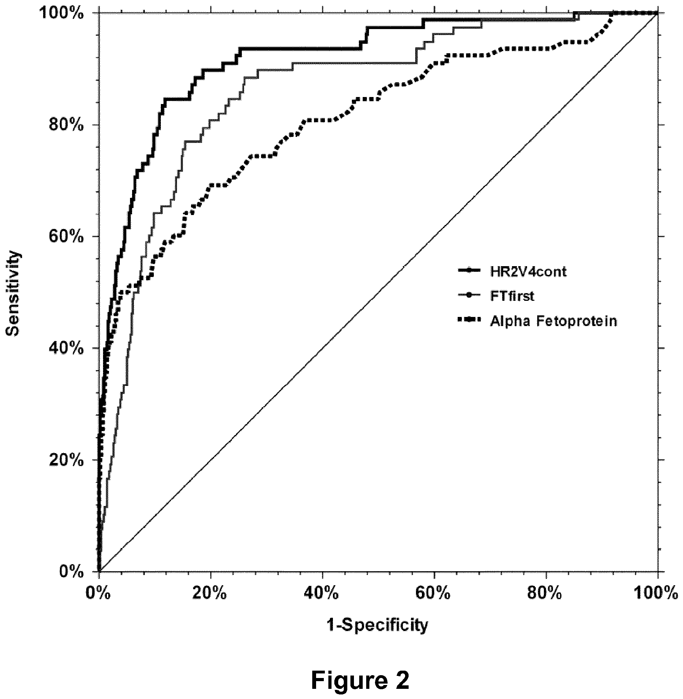

Figure 2: Compared AUROC (Area Under Receiving Operator Curve) for detection of contemporaneous primary liver cancer from function F2-a (HR2V4cont), Fibrotest (FTfirst) and Alpha Fetoprotein.

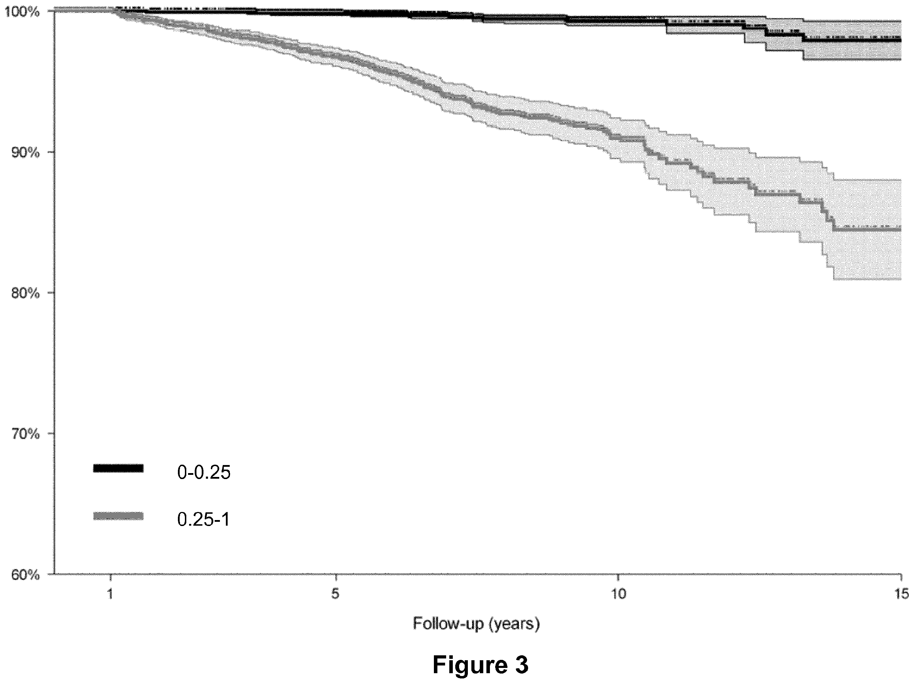

Figure 3: Representation of the percentage of patients without cancer, overtime, depending on their classification, according to the score obtained with the f2-a function. Standard deviation is represented. 0-0.25: patients with a score below 0.25; 0.25-1: patients with a score above or equal to 0.25.

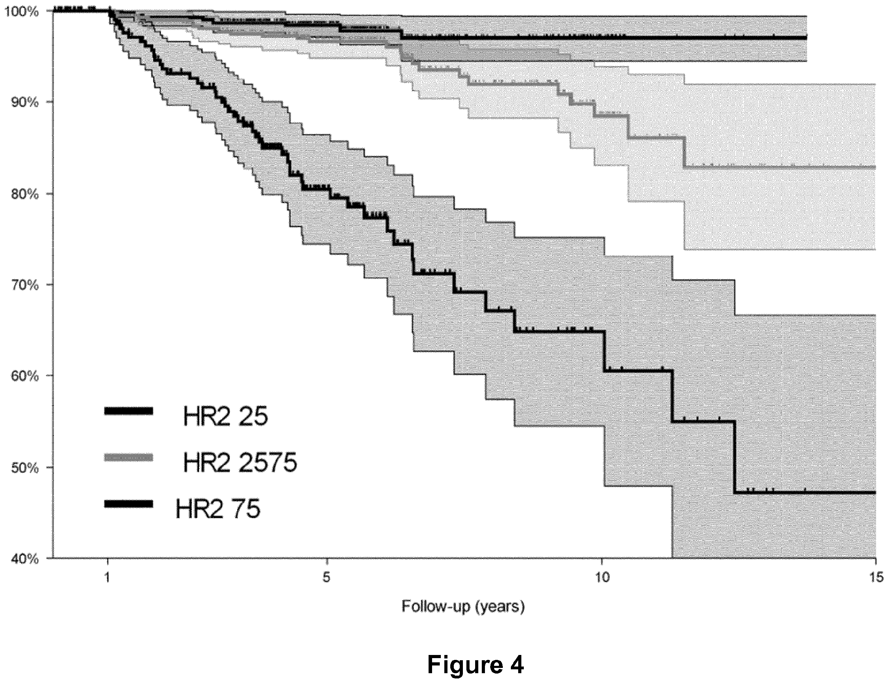

Figure 4: Representation of the percentage of patients without cancer, overtime, depending on their classification, according to the score obtained with the f2-a function. Standard deviation is indicated. HR2 25: patients with score below 0.25; HR2 2575: patients with a score above or equal to 0.25 and below 0.75; HR2 75: patients with a score above or equal to 0.75.

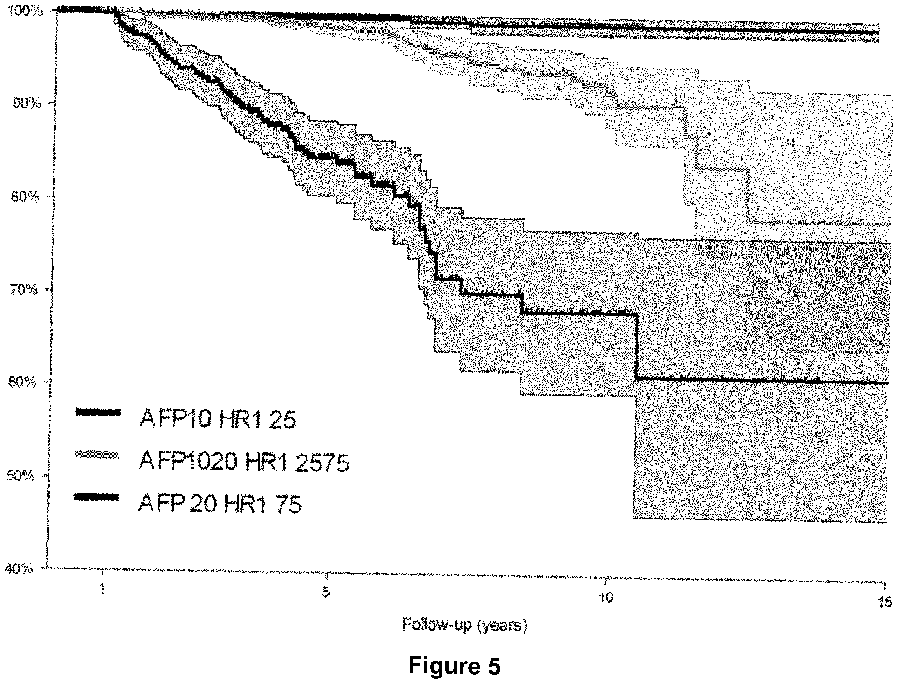

Figure 5: Representation of the percentage of patients without cancer, overtime, depending on their classification, according to the score obtained with the f1-a function and the independent measurement of AFP level. Standard deviation is indicated. AFP10 HR1 25: patients with score below 0.25 and AFP level below 10 $\mu$g/ml; AFp1020 HR1 2575: patients with a score above or equal to 0.25 and below 0.75 and a AFP level between 10 and 20 $\mu$g/ml; AFP20 HR1 75: patients with a score above or equal to 0.75 or AFP level above 20 $\mu$g/ml.

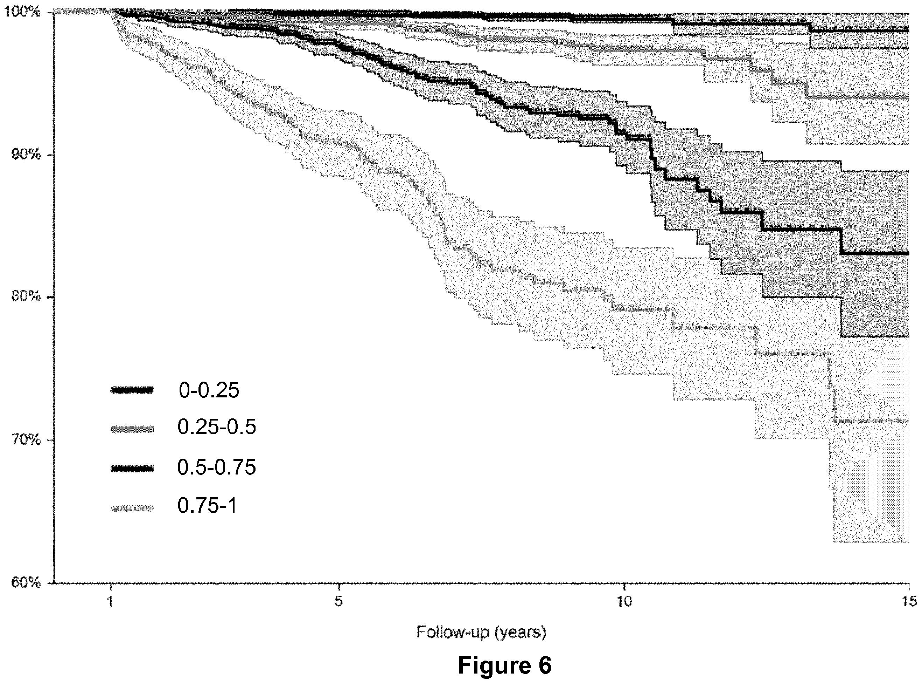

Figure 6: Representation of the percentage of patients without cancer, overtime, depending on their classification (four classes), according to the score obtained with the f1-b function. Standard deviation is indicated. 0-0.25: patients with score below 0.25; 0.25-0.5: patients with a score above or equal to 0.25 and below 0.5; 0.5-0.75: patients with a score above or equal to 0.5 and below 0.75; 0.75-1: patients with a score above or equal to 0.75.

## EXAMPLES

## Example 1. Methods

Functions were obtained on a cohort of 9,925 patients with chronic liver diseases (named pre-inclusion population) who all had a FibroTest measurement (Fibrotest as disclosed in WO 2002/016949). Since the Fibrotest formula is 4.467 x Log(Alpha2Macroglobulin (g/l)) - 1.357 x Log(Haptoglobin (g/l)) + 1.017 x Log(GGT (IU/l)) + 0.0281 x Age (in years) + 1.737 x Log(Bilirubin (pmol/l)) - 1.184 x ApoA1 (g/l) + 0.301 x Sex (female=0, male=1) -5.540, this ensured that measurements of levels of Alpha2Macroglobulin, Haptoglobin, GGT, age (at the time of Fibrotest was performed), Bilirubin, ApoA1 and Sex were available. Furthermore, patients were pre-included only if they had not had previous liver transplantation, and no PLC history.

Primary Liver Cancer was defined as hepatocellular (HCC) or cholangiocarcinoma (CC) according to biopsy, or if missing, Barcelona criteria, or death certificate.

In the construction population (named P0 population), the various mathematical functions, such as f1-a, f1-b and f2-a were obtained by logistic regression, as explained in the specification, using Alpha2Macroglobulin, Haptoglobin, GGT, age (at the time of Fibrotest measurement), Bilirubin, ApoA1 and Sex. Only contemporaneous PLC were taken into account. The performances of functions were assessed using their AUROCs for the diagnostic of contemporaneous PLC.

The prognostic values of the tests were assessed using AUROC and Cox model, in a second population P1 (named longitudinal validation population), with patients who did not develop PLC at least 1year after the baseline test. This ensured that the analysis is not biased by contemporaneous liver cancers, and that the score is a good prognosis score for occurrence of primary liver cancer.

One internal validation population was used, (named P2 population), a subpopulation of P1 with at least 2 repeated tests (two repeated measurement of biochemical markers) to assess intra-patient variability.

A total of 9,700 patients were included in P0 with

- 33.9% Chronic Hepatitis C (CHC),
- 20.5% Chronic Hepatitis B (CHB),
- 10.8% Non Alcoholic Fatty Liver Disease (NAFLD),
- 4.8% Alcoholic Liver Disease (ALD)
- 30.0 % mixed or other causes

Presence of 134 contemporaneous PLC in P0 (129 hepatocellular, 5 cholangiocarcinoma).

A total of 9,791 patients were included in P1 with presence of 225 incident PLC (216 hepatocellular 9 cholangiocarcinoma), observed within 15 years. Taking into account the patients lost during that time span one can calculate a 15 year-survival without PLC (SwC) = 90% (95%CI: 88-92).

A total of 1,773 patients were included in P2 (58 incident PLC with at least 2 repeated tests (7.2 (5.8-6.2) years later), leading to a 15-years SwC 81% (95%CI 72-91)).

### Example 2. Evaluation of the functions

Determination of the diagnosis ability of the function

In P0, HR-Test, a combination of three proteins, one liver function test, age and gender depicted as f1-a, had an AUROC=0.915 (0.889-0.936).

In P1, HR-Test retrieved significant AUROC= 0.828 (0.803-0.850), as well as in P2 for paired cases:= 0.812 (CI 0.772-0.846).

Further data as shown on Figures - Determination of the prognosis ability of the functions

From Figure 1, one can see that a blood test using the same markers (HR1V4) than the ones of Fibrotest (using actually less biochemical markers) is more sensitive and specific than Fibrotest for detecting presence of primary liver cancer (contemporaneous liver cancer), as attested by a higher AUROC.

Figure 2 shows that a blood test using both fibrosis markers as used in Fibrotest and AFP (marker of liver cancer) is more sensitive and specific than these two tests for detecting presence of primary liver cancer (contemporaneous liver cancer), as attested by a higher AUROC. In comparison with FibroTest, AFP is more specific but much less sensitive.

Figure 2 shows the survival without primary liver cancer over time for patients, classified in two classes according to their score with the f1-a function. One can clearly see that a score below 0.25 is indicative of a very little risk of having a primary liver cancer at 5, 10 or 15 years, while the risks increases over time (as soon as one year after the test) for patients with a score equal or above 0.25.

In Figure 3, a test combining both fibrosis markers and a cancer marker (f2-a) has been used to classify the patients in three classes. There is a clear difference between these three classes, and a clear very increased risk of having liver cancer in the following 15 years for patients having a score higher than 0.75 at the time of the test (absolute risk of 50 % at 15 years).

In Figure 5, the f1-a test and independent AFP measurement test have been used, and patients have been classified according to their results for each of these two tests. This figure shows that these two tests can be used together to obtain a risk for the patients to have the primary cancer liver over time. This can be used to identify new cancer liver markers.

In Figure 6, four classes of patients with repeated tests have been determined, according to the score of f1-b. once again, it can be seen that patients with a score below 0.25 have a very little risk to have a cancer in the following 25 years.

All together, these figures show that the methods, functions, tests and scores herein disclosed are more sensitive and specific to detect contemporaneous liver cancers than existing blood tests. They are also predictive of the risk for the patient to have a primary liver cancer over the time. Furthermore, the tests are quantitative, and an increase result is indicative of an increased risk. These tests, functions and scores can be used by themselves, or together with existing liver cancer tests. They further add to the quality of these tests.

**Claims**

1. An *in vitro* method for determining whether a patient has a risk of developing a primary liver cancer, comprising the step of combining the values of biochemical markers as measured in the blood, serum or plasma of the patient through a function, obtaining an end value, comparting the end value to predetermined values, wherein the variation of the end value to the predetermined values indicates the risk of the patient to develop a primary cancer liver.

2. The *in vitro* method of claim 1, wherein the markers are selected from the group consisting of $\alpha$2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1 and carbohydrate deficient transferrin.

3. The *in vitro* method of claim 1 or 2, wherein the function further includes the values measured for at least one liver cancer marker, in particular selected from the group consisting of $\alpha$-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-L-fucosidase (AFU), Des-$\gamma$-carboxyprothrombin (DCP or PIVKA), Osteopontin (OPN), or Human Carbonyl Reductase.

4. The *in vitro* method of any one claim 1 to 3, wherein the function further includes function further includes at least one other variable chosen in the group consisting of gender, age and BMI (Body Mass Index) of the patient.

5. The *in vitro* method of any one of claims 1 to 4, wherein the function is a logistic function obtained by logistic regression.

6. The *in vitro* method of claim 5, wherein the function has been obtained by :

   a) evaluating the presence of liver cancer in a cohort of patients presenting chronic liver disease, wherein the values of blood markers are known for the patients
   b) identifying by unidimensional analysis, the blood markers for which the values differ significantly between the groups of

      i. patients with liver cancer and
      ii. patients without liver cancer

   c) performing a logistic regression analysis to assess and ponder the independent discriminative value of the markers identified in step b) for the occurrence of liver cancer
   d) thereby obtaining the function, by combination of these identified independent factors.

7. The *in vitro* method of any one of claims 1 to 6, wherein the function includes the values measured for Alpha-2-macroglobulin, ApoA1, GGT and Haptoglobin, as well as the values corresponding to the age and sex of the patient.

8. The *in vitro* method of any one of claims 1 to 7, wherein the function is a0 + a1 x Log (A2M, g/l) + a2 x Age (years) + a3 x ApoA1 (g/l) + a4 x Gender (0 for women, 1 for men)+ a5 x Log(GGT, IU/l) + a6 x Log (Hapto, g/l).

9. The *in vitro* method of claim 8, wherein

a) -6 ≤ a0 ≤ -3.4 preferably -5.2 ≤ a0 ≤ -4.7
b) 2.4 ≤ a1 ≤ 4.6 preferably 3 ≤ a1 ≤ 4
c) 0.02 ≤ a2 ≤ 0.07 preferably 0.03 ≤ a2 ≤ 0.07
d) -2.6 ≤ a3 ≤ -0.8 preferably -2.2 ≤ a3 ≤ -1.0
e) -1.5 ≤ a4 ≤ -0.5 preferably -1.1 ≤ a4 ≤ -0.6
f) 0.9 ≤ a5 ≤ 1.9 preferably 1.2 ≤ a5 ≤ 1.6
g) -1.5 ≤ a6 ≤ -0.5 preferably -1.1 ≤ a6 ≤ -0.6.

10. The *in vitro* method of any one of claims 1 to 9, wherein the function is chosen in the group consisting of

    a) F1-a = -4.819 + 3.673 x LogA2M (g/L) + .053 x Age (years) - 1.983 x ApoA1 (g/L) - 1.122 x Sex (0 for women, 1 for men) + 1.603 x LogGGT (IU/L) - 0.834 x LogHapto (g/L), and
    b) F1-b = -4.982 +3.713 x LogA2m (g/L) + 0.0473 x Age (years) -1.133 x ApoA1 (g/L) - 0.791 x Sex (0 for women, 1 for men) + 1.343 x LogGGT (IU/L) - 1.062 x LogHapto (g/L)

11. The *in vitro* method of claim 10, wherein the predetermined value is 0.25, and wherein the patient has an increased risk to develop a primary liver cancer if the end result is higher or equal to 0.25.

12. The *in vitro* method of any one of claims 1 to 7, wherein the function is a0 + a1 x Log (A2M, g/l) + a2 x Age (years) + a3 x ApoA1 (g/l) + a4 x Gender (0 for women, 1 for men)+ a5 x Log(GGT, IU/l) + a6 x Log (Hapto, g/l) + a7 x Log (AFP, μg/L)

13. The *in vitro* method of claim 12, wherein

    a) -7 ≤ a0 ≤ -5.5 preferably -6.5 ≤ a0 ≤ -6
    b) 2.2 ≤ a1 ≤ 3.2 preferably 2.5 ≤ a1 ≤ 2.9
    c) 0.02 ≤ a2 ≤ 0.06 preferably 0.04 ≤ a2 ≤ 0.05
    d) -1.65 ≤ a3 ≤ -1.25 preferably -1.55 ≤ a3 ≤ -1.35
    e) -0.3 ≤ a4 ≤ -0.22 preferably -0.28 ≤ a4 ≤ -0.24
    f) 1.25 ≤ a5 ≤ 1.85 preferably 1.45 ≤ a5 ≤ 1.65
    g) -0.75 ≤ a6 ≤ -0.55 preferably -0.7 ≤ a6 ≤ -0.6
    h) 1.3 ≤ a7 ≤ 1.9 preferably 1.5 ≤ a7 ≤ 1.8

14. The *in vitro* method of any one of claims 12 or 13, wherein the function is F2-a = -6.214 + 2.713 LogA2m (g/L) + 0.0447 x Age (years) - 1.451 x ApoA1 (g/L) - 0.260 x Sex (0 for women, 1 for men) + 1.557 x LogGGT (IU/L) -0.633 x LogHapto (g/L) + 1.662 x LogAFP (μg/L).

15. The *in vitro* method of any one of claims 1 to 4, wherein the function is a multivariate Cox function.

16. A method for obtaining a function that can assist a physician to determine whether a patient has a risk of having primary liver cancer wherein said function combines the values of the concentration of biochemical markers in the blood / serum or plasma of said patient and optionally the age, gender and BMI, comprising the steps of:

    a) evaluating the presence of liver cancer after a given duration of time in a cohort of patients, wherein the values of the blood markers variables used in the function are known for the patients at the beginning of the duration of time,
    b) identifying by unidimensional analysis, the biochemical markers for which the measured value differs significantly between the group of patients in which cancer has occurred and the group of patients in which cancer has not occurred
    c) performing a logistic regression analysis to assess the independent discriminative value of these markers identified in step b), and optionally the age, gender and BMI of the patient, for the occurrence of primary liver cancer
    d) obtaining the logistic function by combination of these identified independent factors.

17. The method of claim 16, wherein the biochemical markers of step b) are selected from the group consisting of α2-macroglobulin (A2M), GGT (gammaglutamyl transpeptidase), haptoglobin, apolipoprotein A-I (apoA1), bilirubin, alanine transaminases (ALT), aspartate transaminases (AST), triglycerides, total cholesterol, fasting glucose, γ-globulin, albumin, α1-globulin, α2-globulin, β-globulin, IL10, TGF-β1, apoA2, apoB, cytokeratin 18, platelets number, prothrombin level, hyaluronic acid, urea, N-terminal of type III pro-collagen, tissue inhibitor metalloproteinase type-1 (TIMP-1), type IV collagen (Coll IV), osteoprotegerin, miRNA122, cytokeratin-18, serum amyloid A (SAA), alpha-1-antitrypsin (isoform 1), fructose-bisphosphate aldolase A, Fructose-bisphosphate aldolase B, fumarylacetoacetase, transthyretin, PR02275, C-reactive protein (isoform 1), leucine-rich alpha-2-glycoprotein, serpin A11, DNA-directed RNA polymerase I subunit RPA1, obscurin (isoform 1), alpha-skeletal muscle actin, aortic smooth muscle actin, alkaline phosphatase, uncharacterized protein C22orf30 (isoform 4), serum amyloid A2 (isoform a), apolipoprotein C-III, apolipoprotein E, apolipoprotein A-II, polymeric immunoglobulin receptor, von Willebrand factor, aminoacylase-1, G-protein coupled receptor 98 (isoform 1), paraoxonase/arylesterase 1, complement component C7, hemopexin, complement C1q subcomponent, paraoxonase/lactonase 3, complement C2 (fragment), versican core protein (isoform Vint), extracellular matrix protein 1 (isoform 1), E3 SUMO-protein ligase RanBP2, haptoglobin-related protein (isoform 1), adiponectin, retinol binding protein, ceruloplasmin, alpha 2 antiplasmin, antithrombin, thyroxin binding protein, protein C, alpha 2lipoprotein, tetranectin, fucosylated A2M, fucosylated haptoglobin, fucosylated apoA1, carbohydrate deficient transferrin, $\alpha$-fetoprotein (AFP), fucosylated AFP, HSP27 (heat shock protein), HSP70, Glypican-3 (GPC3), squamous cell carcinoma antigen (SCCA) and in particular SCCA-IgM IC which is a circulating immune complex composed of SCCA and IgM, Golgi protein 73 (GP73), $\alpha$-L-fucosidase (AFU), Des-$\gamma$-carboxyprothrombin (DCP or PIVKA), Osteopontin (OPN), and Human Carbonyl Reductase.

**Figure 1**

Figure 2

Figure 3

**Figure 4**

**Figure 5**

Follow-up (years)

**Figure 6**

Europäisches
Patentamt

European
Patent Office

Office européen
des brevets

# EUROPEAN SEARCH REPORT

Application Number

EP 17 30 6403

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEI-HSUAN LEE ET AL: "Prediction models of long-term Cirrhosis and hepatocellular carcinoma risk in chronic hepatitis B patients: Risk scores integrating host and virus profiles : Hepatology", HEPATOLOGY, vol. 58, no. 2, 29 July 2013 (2013-07-29), pages 546-554, XP055442303, ISSN: 0270-9139, DOI: 10.1002/hep.26385 * abstract, page 548 * | 1,16,17 | INV. G01N33/574 G16H50/30 |
| X | CHI-PANG WEN ET AL: "Hepatocellular Carcinoma Risk Prediction Model for the General Population: The Predictive Power of Transaminases", JOURNAL OF THE NATIONAL CANCER INSTITUTE, vol. 104, no. 20, 16 October 2012 (2012-10-16), pages 1599-1611, XP055442306, GB ISSN: 0027-8874, DOI: 10.1093/jnci/djs372 * abstract, page 1600, right-hand column, section "statistical analysis" - page page 1602, left-hand column. * | 1,16,17 | |
| X A | US 2017/032099 A1 (CALES PAUL [FR] ET AL) 2 February 2017 (2017-02-02) * abstract, paragraphs [0036]-[0047], [0054], [0089], [0092], [0098]-[0100], example 2. * | 1-7, 15-17 8-14 | TECHNICAL FIELDS SEARCHED (IPC) G01N G16H |
| Y | EP 2 600 266 A1 (BIOPREDICTIVE [FR]; ASSIST PUBL HOPITAUX DE PARIS [FR]) 5 June 2013 (2013-06-05) * claims 1, 2 * | 1,2,4-7, 15 | |

-/--

The present search report has been drawn up for all claims

| Place of search | Date of completion of the search | Examiner |
|---|---|---|
| Munich | 6 February 2018 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 17 30 6403

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MULTIVIRC GROUP IMBERT-BISMUT ET AL: "Biochemical markers of liver fibrosis in patients with hepatitis C virus infection: a prospective study", THE LA, THE LANCET PUBLISHING GROUP, GB, vol. 357, no. 9262, 7 April 2001 (2001-04-07), pages 1069-1075, XP005061312, ISSN: 0140-6736, DOI: 10.1016/S0140-6736(00)04258-6 * abstract, page 1070 section "statistical analysis", page 1071, left-hand column, table 2. * ----- | 1,2,4-7, 15 | |
| Y | EP 2 799 876 A2 (CHANCELLORS MASTERS AND SCHOLARS OF THE UNIVERSITY OF OXFORD [GB]) 5 November 2014 (2014-11-05) * paragraph [0005] * ----- | 1,2,4-7, 15 | |
| | | | **TECHNICAL FIELDS SEARCHED (IPC)** |

The present search report has been drawn up for all claims

| Place of search | Date of completion of the search | Examiner |
|---|---|---|
| Munich | 6 February 2018 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 30 6403

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2018

| Patent document cited in search report | | | Publication date | Patent family member(s) | | | Publication date |
|---|---|---|---|---|---|---|---|
| US 2017032099 | A1 | | 02-02-2017 | CN | 106462666 | A | 22-02-2017 |
| | | | | EP | 2930515 | A1 | 14-10-2015 |
| | | | | EP | 3129787 | A1 | 15-02-2017 |
| | | | | US | 2017032099 | A1 | 02-02-2017 |
| | | | | WO | 2015155259 | A1 | 15-10-2015 |
| EP 2600266 | A1 | | 05-06-2013 | CN | 104094264 | A | 08-10-2014 |
| | | | | EP | 2600266 | A1 | 05-06-2013 |
| | | | | EP | 2786287 | A1 | 08-10-2014 |
| | | | | US | 2014329260 | A1 | 06-11-2014 |
| | | | | WO | 2013079711 | A1 | 06-06-2013 |
| EP 2799876 | A2 | | 05-11-2014 | CA | 2761692 | A1 | 25-11-2010 |
| | | | | CN | 102460174 | A | 16-05-2012 |
| | | | | CN | 105866423 | A | 17-08-2016 |
| | | | | EP | 2430451 | A1 | 21-03-2012 |
| | | | | EP | 2799876 | A2 | 05-11-2014 |
| | | | | ES | 2527455 | T3 | 23-01-2015 |
| | | | | HK | 1166368 | A1 | 09-06-2017 |
| | | | | JP | 5813630 | B2 | 17-11-2015 |
| | | | | JP | 5897638 | B2 | 30-03-2016 |
| | | | | JP | 2012526980 | A | 01-11-2012 |
| | | | | JP | 2014197006 | A | 16-10-2014 |
| | | | | JP | 2016138891 | A | 04-08-2016 |
| | | | | KR | 20120041175 | A | 30-04-2012 |
| | | | | US | 2010291602 | A1 | 18-11-2010 |
| | | | | US | 2015105293 | A1 | 16-04-2015 |
| | | | | US | 2016202274 | A1 | 14-07-2016 |
| | | | | WO | 2010133967 | A1 | 25-11-2010 |

EPO FORM P0459

For more details about this annex : see Official Journal of the European Patent Office, No. 12/82

## REFERENCES CITED IN THE DESCRIPTION

**Patent documents cited in the description**

- WO 2002016949 A
- WO 2010149767 A
- WO 200610357 A
- WO 2006082522 A
- WO 2003073822 A
- WO 2011039321 A
- WO 2005116901 A
- WO 2010058295 A
- WO 2010097472 A
- WO 0216949 A

**Non-patent literature cited in the description**

- **ZHAO et al.** *Mol Clin Oncol.,* July 2013, vol. 1 (4), 593-598
- **SALLOOM.** *Int J Health Sci (Qassim).,* January 2016, vol. 10 (1), 121-136
- **TARA BEHNE ; COPUR.** *International Journal of Hepatology,* vol. 2012, 7